# EUROPEAN PATENT SPECIFICATION

(11) **EP 2 844 126 B1**
(45) Date of publication and mention of the grant of the patent: **19.07.2017**
(21) Application number: 12873165.0
(22) Date of filing: 30.03.2012
(51) Int. Cl.: A61B 18/22, A61B 1/005, A61B 18/00, A61B 1/307, A61M 25/01

(54) **SURGICAL INSTRUMENT HANDLE WITH A CAM-ACTUATING SYSTEM**
GRIFF FÜR CHIRURGISCHES INSTRUMENT MIT EINEM OPERATIONSINSTRUMENTENGRIFF MIT EINEM EXZENTERBETÄTIGENDEN SYSTEM
MANCHE D'INSTRUMENT CHIRURGICAL COMPRENANT UN SYSTÈME DE MISE EN MARCHE À CAME

(43) Date of publication of application: 11.03.2015
(73) Proprietor: Synergetics, Inc., O'Fallon, MO 63368 (US)
(72) Inventor: LACONTE, Matthew, O'Fallon, MO 63368 (US); HANLON, Matthew, O'Fallon, MO 63368 (US)
(74) Representative: Wallis, Naomi Rachel
(86) International application number: PCT/US2012/031495
(87) International publication number: WO 2013/147857

(56) References cited:
- EP-A2- 0 531 710
- WO-A2-2004/091380
- US-A- 5 483 952
- US-A- 5 490 409
- US-A1- 2011 144 630
- US-A1- 2011 282 190
- US-A1- 2012 029 354
- US-B1- 6 443 968

## Description

### FIELD

This document relates generally to surgical instruments and in particular to surgical instrument handles having a cam-actuating system for operating a surgical instrument.

### PRIOR ART

US2011/44630 discloses a fiber optic device including an optical fiber passing through a handpiece that defines an internal passageway with an apertured endwall at a distal end of the passageway. A portion of the optical fiber is disposed within the passageway, and a stop member, such as a rigid sheath, within the passageway is attached to the optical fiber. An indexing member releasably engages the rigid sheath to maintain the rigid sheath at a predetermined position within the passageway. The rigid sheath preferably contains a series of index positions for incrementally adjusting the position of the sheath within the passageway. The rigid sheath is slidably mounted in the passageway and is configured to interfere with the endwall when the rigid sheath is released for movement within the internal cavity to its final position within the passageway.

### SUMMARY

A surgical instrument handle of the present invention includes a handle body, a rotating member pivotally engaged to the handle body for rotation in first or second rotational directions wherein the rotating member defines a cam-actuating surface, a locking member configured to engage the cam-actuating surface of the rotating member, and a reciprocating member engaged to the locking member, wherein the rotational action of the rotating member in a first rotational direction causes lateral movement of the reciprocating member in a first longitudinal direction as the locking member travels along the cam-actuating surface of the rotating member and a rotational action of the rotating member in a second rotational direction causes lateral movement of the reciprocating member in a second longitudinal direction as the locking member travels along the cam-actuating surface of the rotating member, wherein the cam-actuating surface is defined along an interior surface of the rotating member, wherein the interior surface defines a slot having a first end and a second end, wherein the cam-actuating surface is configured to produce a cam action by the locking member as the locking member moves between the first end and the second end of the cam-actuating surface that translates rotational movement of the rotating member to longitudinal movement of the reciprocating member.

Another surgical instrument handle contemplated herein may include a handle body, a rotating member pivotally engaged to the handle body, a locking member defining a cam-actuating surface and in sliding engagement with the rotating member, and a reciprocating member engaged to the locking member, wherein the rotational action of the rotating member in a first rotational direction causes lateral movement of the reciprocating member in a first longitudinal direction as the rotating member travels along the cam-actuating surface of the locking member and a rotational action of the rotating member in a second rotational direction causes lateral movement of the reciprocating member in a second longitudinal direction as the rotating member travels along the cam-actuating surface of the locking member.

A method of manufacturing a surgical instrument handle contemplated herein may include:
forming a handle body;
forming a rotating member defining a cam-actuating surface and pivotally engaging the rotating member to the handle body;
engaging a locking member to the cam-actuating surface of the rotating member such that the locking member travels along the cam-actuating surface of the rotating member when the rotating member is rotated; and
coupling the locking member to a reciprocating member such that the travel of the locking member along the cam-actuating surface of the rotating member causes the reciprocating member to move in longitudinal direction.

Another method of manufacturing a surgical instrument handle contemplated herein may include:
forming a handle body;
pivotally engaging a rotating member to the handle body;
forming a locking member defining a cam-actuating surface and engaging the rotating member to the cam-actuating surface of the locking member; and
engaging the locking member to a reciprocating member;
wherein the travel of the rotating member along the cam-actuating surface of the locking member causes the reciprocating member to move in a longitudinal direction.

Additional objectives, advantages and novel features will be set forth in the description which follows or will become apparent to those skilled in the art upon examination of the drawings and detailed description which follows.

### BRIEF DESCRIPTION OF THE DRAWINGS

FIG. **1** is a side view of a surgical instrument handle;
FIG. **2** is a cross-sectional view of the surgical instrument handle taken along line **2-2** of FIG. **1**;
FIG. **3** is an enlarged view showing a rotating member with a cam-actuating surface for the surgical instrument handle of FIG. **2** operatively engaged to a locking member;
FIG. **4** is an end view of the surgical instrument handle;
FIG. **5** is a top view of the surgical instrument handle;
FIG. **6** is an enlarged view of the surgical instrument handle of FIG. **3**;
FIG. **7** an exploded view of the surgical instrument handle illustrating the connection of the surgical instrument handle to an external connector through a cable;
FIGS. **8A-C** are cross-sectional views of the surgical instrument handle showing a sequence of operation; and
FIG. **9** is an enlarged exploded view of the surgical instrument handle of FIG. **7**;
FIG. **10** is a cross-sectional view of a tube and wire arrangement of the surgical instrument handle taken along line **10-10** of FIG. **2**;
FIG. **11** is another cross-sectional view of the tube and wire arrangement of the surgical instrument handle taken along line **11-11** of FIG. **2**;
FIG. **12** is a top view of another surgical instrument handle contemplated herein;
FIG. **13** is a cross-sectional view of the surgical instrument handle taken along line **13-13** of FIG. **12**; and
FIG. **14** is an enlarged view of the surgical instrument handle of FIG. **13** showing a locking member with a cam-actuating surface operatively engaged to a pivotal rotating member.

Corresponding reference characters indicate corresponding elements among the view of the drawings. The headings used in the figures should not be interpreted to limit the scope of the claims.

### DETAILED DESCRIPTION

As described herein, a surgical instrument handle operates a surgical instrument using a cam-actuating system that translates rotational movement of one structural member into longitudinal movement of another structural member.

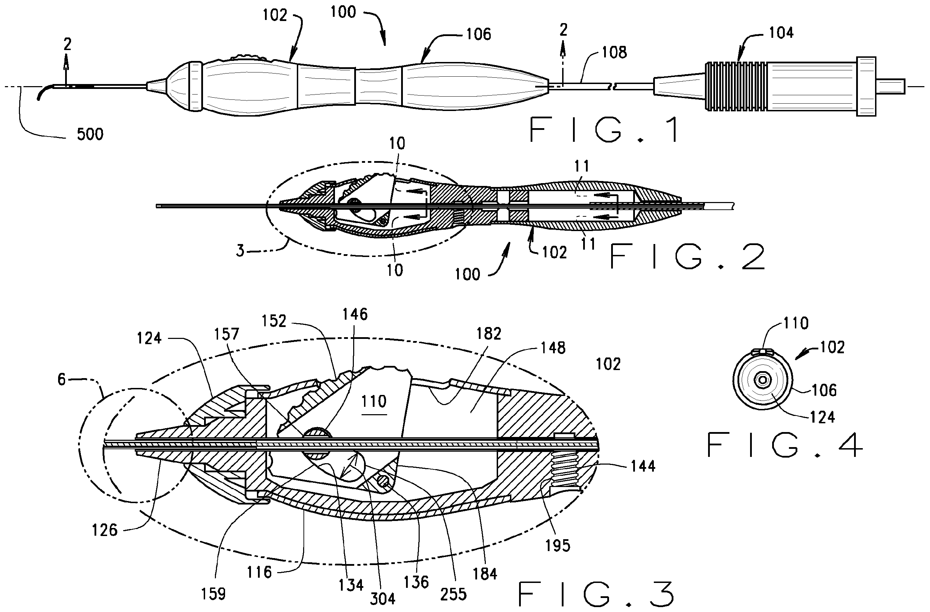

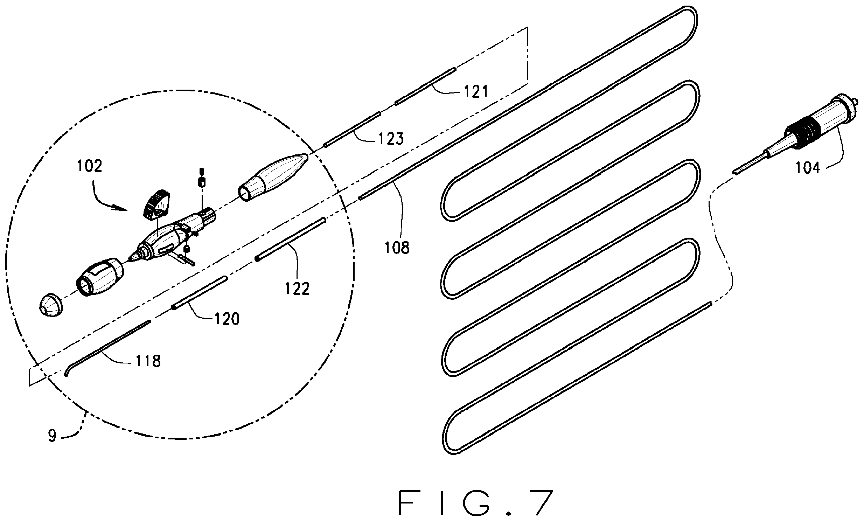

Referring to the drawings, various embodiments of a surgical instrument are illustrated and generally indicated as **100** and **200** in FIGS. **1** - **14****.** In one embodiment shown in FIG. **1**, the surgical instrument **100** may be a laser probe apparatus having a surgical instrument handle **102** operatively connected through a cable **108**, such as a fiber-optic cable, to a connector **104** for connecting the surgical instrument handle **102** to a source of power for operating the surgical instrument **100.** In some embodiments, the surgical instrument handle **102** includes an elongated handle body **106** configured for one-handed operation by an individual in which rotation of a rotating member **110** (FIG. **3**) that is pivotally mounted to the handle body **106** causes a retraction or extension action for operating the surgical instrument **100** as shall be described in greater detail below.

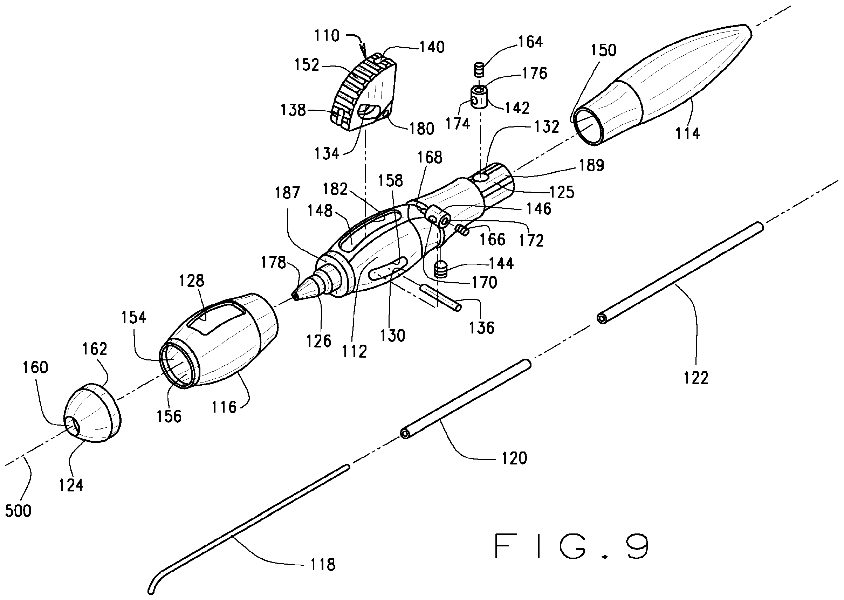

Referring to FIG. **9**, one embodiment of the surgical instrument handle **102** includes an inner casing **112** having a proximal end **189** coupled to a rear casing **114** and a distal end **187** coupled to an outer casing **116.** The outer casing **116** defines a longitudinally-extending top slot **128** that communicates with a chamber **148** defined within the inner casing **112** when the outer casing **116** is coupled to the inner casing **112.** The outer casing **116** also defines a distal opening **154** configured to engage the proximal end **162** of a nose cone casing **124** having a nose cone opening **160** when the surgical instrument handle **102** is assembled. When the surgical instrument handle **102** is fully assembled, the inner casing **112**, rear casing **114** and outer casing **116** collectively encase a hollow reciprocating member **120** in which a memory-shaped hollow tube **118** is coaxially disposed within the reciprocating member **120** that extends longitudinally through the handle body **106** substantially along longitudinal axis **500** (FIG. **1**). In one embodiment, the memory-shaped hollow tube **118** is made from a memory-shaped material that may be bent and maintain the bent orientation. The reciprocating member **120** is operative to move between a fully extended position and a fully retracted position. In addition, the reciprocating member **120** is slidably engaged relative to the memory-shaped hollow tube **118** such that the reciprocating member **120** moves relative to the memory-shaped hollow tube 118, which is fixed in position. As shown, the reciprocating member **120** is extendable through a tip opening **178** of a nose cone fitting **126** attached to the inner casing **112** of the handle body **106.**

As further shown, the inner casing **112** includes a rear portion **125** that defines a conduit **132** configured to receive a first locking member **142**, which engages the memory-shaped hollow tube **118.** In some embodiments, the memory-shaped hollow tube **118** may be nickel titanium, such as NITINOL™, or any other type of metallic material that may be bent and maintain a particular bent orientation. Specifically, the first locking member **142** defines an axial conduit **176** configured to receive a set screw **164** that communicates with a longitudinal conduit **174** configured to receive a portion of the memory-shaped hollow tube **118.** When inserted into the axial conduit **174** the set screw **164** engages the memory-shaped hollow tube **118** such that the hollow tube **118** is fixedly secured in place relative to the inner casing **112** of the handle body **106**.

Referring to FIGS. **3** and **9**, when the handle body **106** is assembled a rotating member **110**, such as a wheel or lever, is pivotally engaged to the inner casing **112** of the handle body **106** to permit an individual to actuate the surgical instrument handle **102**. The rotating member **110** is pivotally engaged to the inner casing **112** through a pivot pin **136** inserted through the pin hole **180** formed through a bearing surface **184** of the rotating member **110** in a "slip fit" fashion that allows the rotating member **110** to pivot about an axis defined by the pin hole **180**. In some embodiments, the inner casing **112** defines a side slot **158** configured to permit access to the rotating member **110** when engaging the locking member **146** to a curved slot **134** defined by the rotating member **110** during assembly of the surgical instrument handle **102**. The bearing surface **184** provides a load bearing structure for the rotating member **110**. The rotating member **110** defines a distal clearing notch **138** and a proximal clearing notch **140** that permit the rotating member **110** to clear the inner surface **182** of the chamber **148** when rotated in the most distal and proximal positions. The inner casing **112** further defines an aperture **130** configured to permit access to the rotating member **110** when inserting the pivot pin **136** through the pin hole **180**.

In some embodiments, the rotating member **110** may define a kidney-shaped slot **134** that provides a cam-actuating surface configured to engage a locking member **146** which travels in sliding engagement along the kidney-shaped slot **134** when the rotating member **110** is rotated in either a clockwise direction or counter-clockwise direction. The kidney-shaped slot **134** may have a generally curved, non-linear configuration defining a first end **155**, a second end **157** and a mid-point location **159** in which the width **304** of the slot **134** between any opposite lateral points remains substantially the same. As such, rotation of the rotating member **110** in either the clockwise or counter-clockwise rotational directions causes the locking member **146** to travel along the kidney-shaped slot **134** such that the tangential angle defined at each point of contact between the locking member **146** and the kidney-shaped slot **134** remains at about a 45 degree angle. In some embodiments, the tangential angle defined at the various points of contact between the locking member **146** and the kidney-shaped slot **134** may range between 35-50 degrees. In some embodiments, the rotating member **110** may be pivoted in a range of 90 degrees in either the clockwise and counter-clockwise directions. The rotating member **110** may further include a gripping surface **152** formed along the top peripheral portion of the rotating member **110**, which defines a plurality of protrusions configured to engage the individual's thumb when rotating the rotating member **110** as shall be discussed in greater detail below.

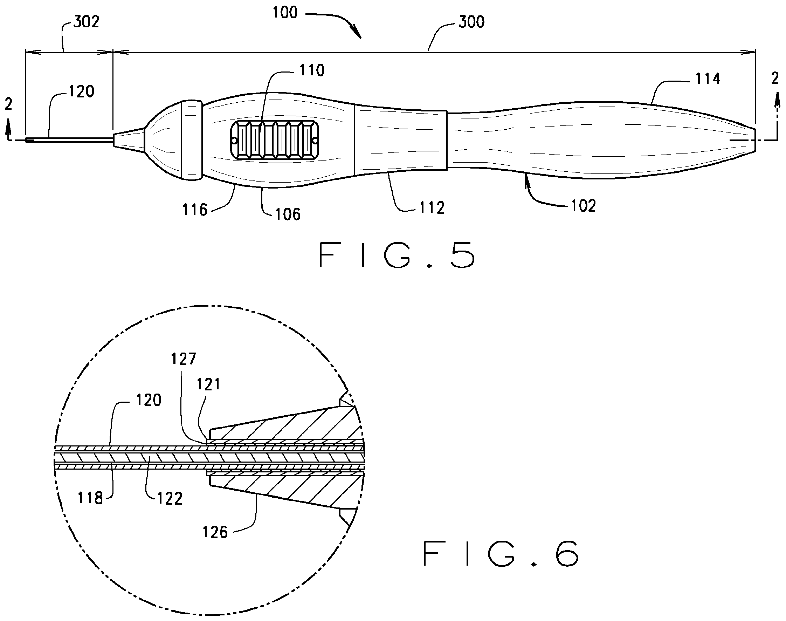

Referring to FIGS. **6****,** **7****,** **9** and **10**, in some embodiments the distal portion of a fiber optic cable **122** may be coaxially disposed within the memory-shaped hollow tube **118** such that the orientation of the free end of the fiber optic cable **122** is set by the particular orientation of the memory-shaped hollow tube **118**. In one arrangement, the memory-shaped hollow tube **118** may be coaxially disposed within the reciprocating member **120**, which is capable of a sliding longitudinal movement relative to the memory-shaped hollow tube **118** in which the memory-shaped hollow tube **118** is fixed in position by the first locking member **142** relative to the reciprocating member **120**. The hollow reciprocating member **120** may be coaxially disposed within a bearing tube **121** along longitudinal axis **500**. In one aspect, the reciprocating member **120** may be engaged to the bearing tube **121**, which is fixedly coupled to the handle body **106**. As such, movement of the bearing tube **121** causes concurrent longitudinal movement of the reciprocating member **120** along longitudinal axis **500**. In one embodiment, the reciprocating member **120** may be **20**- gauge in size, although other gauges may be used in the construction of the reciprocating member **120**. In an alternative embodiment, a bushing **127** (FIG. **6**) may be coaxially disposed within the bearing tube **121** and surround the reciprocating member **120** in order to accommodate different coaxial arrangements of the hollow reciprocating member **120**, bearing tube **121**, and a memory-shaped hollow tube **118**.

As shown in FIG. **3**, the handle body **106** defines an axial conduit **195** in communication with a portion of the bearing tube **121** that extends longitudinally through the axial conduit **195**. The axial conduit **195** is configured for threaded engagement with a tension-setting screw **144** that applies tension against the bearing tube **121**, which allows the user to set the desired level of tension felt when manipulating the rotating member **110**.

Referring to FIGS. **3**, **7** and **9**, the bearing tube **121** is configured to be inserted into a longitudinal conduit **170** defined by the second locking member **146**. The second locking member **146** also defines an axial conduit **172** configured to receive a set screw **166** adapted to engage the bearing tube **121**. In assembly, the second locking member **146** is in sliding engagement with the cam-actuating surface of the kidney-shaped slot **134** such that clockwise or counter-clockwise rotation of the rotating member **110** in a pivoting or rotating motion causes the second locking member **146** to travel along the cam-actuated surface of the kidney-shaped slot **134** in a cam action. The travel of the second locking member **146** along the cam-actuating surface of the kidney-shaped slot **134** translates the pivoting or rotational movement of the rotating member **110** into longitudinal movement of the bearing tube **121**, which is fixedly attached to the reciprocating member **120**, thereby driving the reciprocating member **120** in either the proximal or distal directions when the rotating member **110** is manipulated in a clockwise or counter-clockwise direction, respectively. In one embodiment, manipulation of the rotating member **110** in a clockwise direction causes the reciprocating member **120** to be extended outwardly from the handle body **106** in a distal direction relative to the memory-shaped hollow tube **118**, thereby causing the memory-shaped hollow tube **118** to be fully disposed within the reciprocating member **120**. Conversely, movement of the rotating member **110** in a counter-clockwise direction causes the reciprocating member **120** to be retracted inwardly into the handle body **106** in a proximal direction relative to the memory-shaped hollow tube **118**, thereby causing the memory-shaped hollow tube **118** to be exposed. In other embodiments, the memory-shaped hollow tube **118** may move while the reciprocating member remains fixed in position relative to the surgical instrument handle **102**.

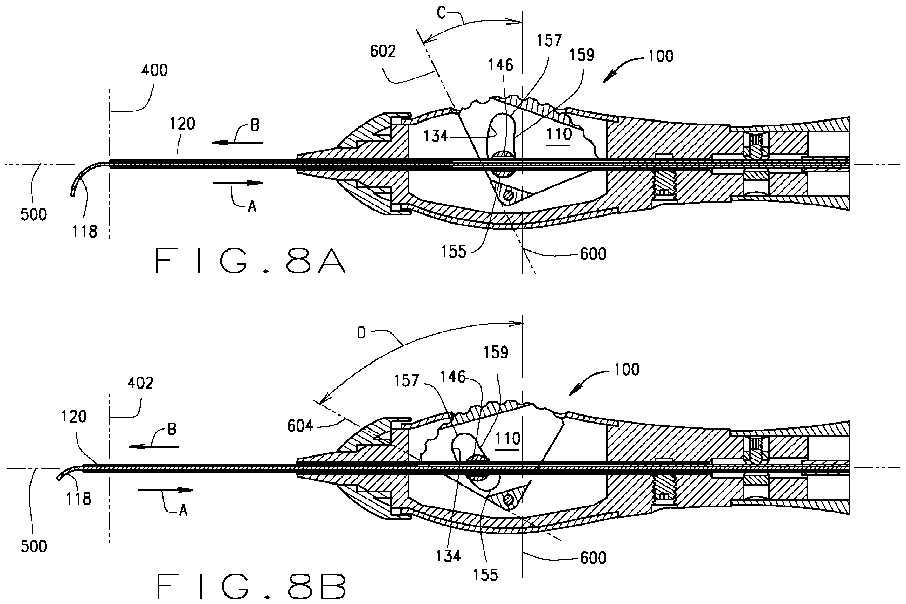

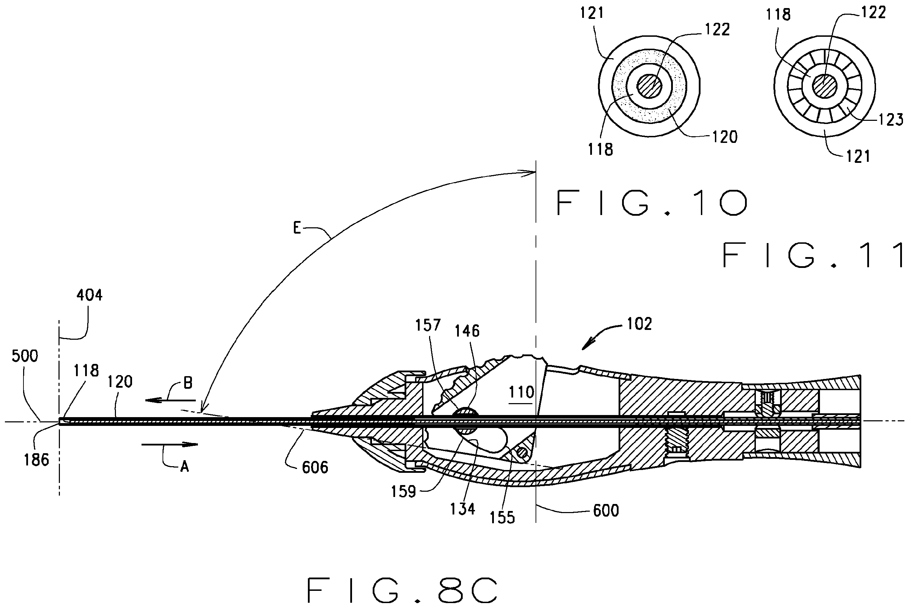

FIGS. **8A-8C** show the sequence of the second locking member **146** in sliding engagement along the cam-actuating surface defined by the kidney-shaped slot **134** as the rotating member **110** is rotated as described above. In FIG. **8A**, the reciprocating member **120** is shown in a fully retracted position such that the memory-shaped hollow tube **118** extends outwardly from the reciprocating member **120**. In this position, the second locking member **146** is located at the first end **155** of the kidney-shaped slot **134** and the reciprocating member **120** is located at a first position **400**. In the fully retracted position, the rotating member **110** is positioned along a plane **602**, which forms an angle **B** with longitudinal plane **600**. In FIG. **8B**, the reciprocating member **120** is shown in a mid-retraction position such that less of the memory-shaped hollow tube **118** extends outwardly from the reciprocating member **120** than in the fully retracted position of the reciprocating member **120** as the rotating member **110** is rotated in a counter-clockwise direction. In this position, the second locking member **146** is located at the mid location **159** of the kidney-shaped slot **134** such that the reciprocating member **120** extends to a second position **402**. In the mid retraction position, the rotating member **110** is positioned along a plane **604**, which forms an angle **C** with longitudinal plane **600**. In FIG. **8C**, the reciprocating member **120** is shown in a fully extended position such that the memory-shaped hollow tube **118** is fully disposed within the reciprocating member **120** as the rotating member **110** has been further rotated in the counter-clockwise direction. In this position, the second locking member **146** is positioned at the second end **157** of the kidney-shaped slot **134** and the reciprocating member **120** extends to a third position **404**. In the fully extended position, the rotating member **110** is positioned along a plane **606**, which forms an angle **D** with longitudinal plane **600.**

Conversely, when the rotating member **110** is rotated in the clockwise direction, the reciprocating member **120** may be moved from the fully extended position of FIG. **8C** when the memory-shaped hollow tube **118** is fully disposed within the reciprocating member **120** to the fully retracted position of FIG. **8A** when the memory-shaped hollow tube **118** extends fully outward from the reciprocating member **120**.

Referring to FIGS. **3** and **11**, in some embodiments a back tube **123** may be coaxially disposed within a portion of the bearing tube **121** proximal or adjacent to the reciprocating member **120**. The back tube **123** may be capable of a sliding engagement with the bearing tube **121**, which extends substantially the longitudinal length of the handle body **106**. A portion of the memory-shaped hollow tube **118**, proximal or adjacent to the reciprocating member **120** may be coaxially disposed within the back tube **123** and surround the fiber optic cable **122**. However, in other embodiments

Referring to FIG. **5**, in one embodiment the handle body **106** may have a length **300** of 105 mm and the reciprocating member **120** may extend a length **302** of 32 mm from the handle body **106** when in the fully extended position. However, in other embodiments the lengths **300** and **302** may vary in dimension.

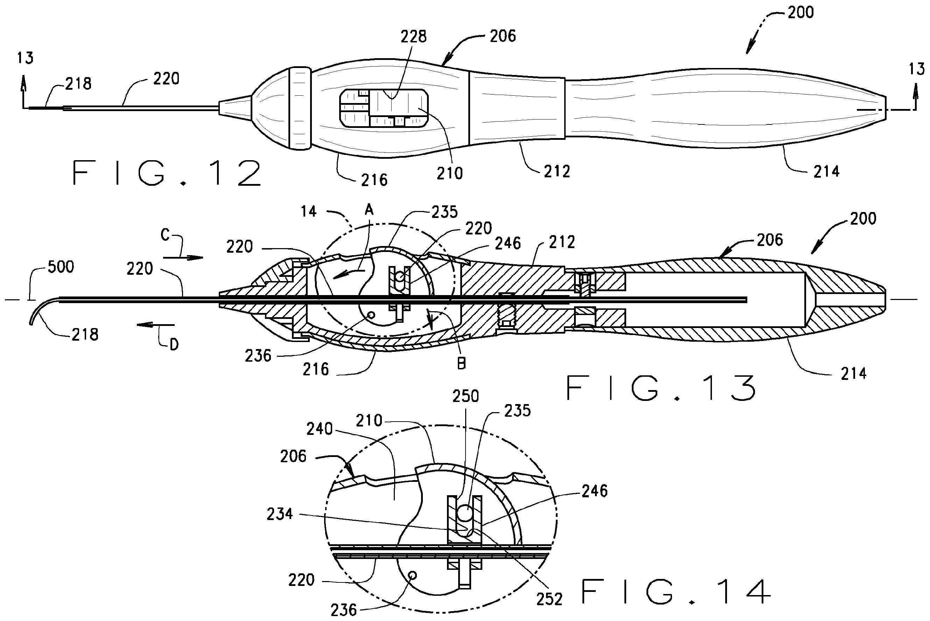

Referring to FIGS. **12**, **13** and **14**, another surgical instrument, designated **200**, is contemplated herein. The surgical instrument **200** includes a surgical instrument handle **202** having a handle body **206** that has an identical or substantially similar configuration to handle body **106** in which the handle body **206** includes an inner casing **212** coupled to an outer casing **216** and a rear casing **214**. The outer casing **216** defines a longitudinal slot **228** having a rotating member **210**, such as a wheel or lever, which extends partially outward from the longitudinal slot **228**. The rotating member **210** is pivotally engaged to the handle body **206** through a pivot pin **236** that allows the rotating member **210** to pivot or rotate in either a clockwise direction or counter-clockwise direction within a chamber **240** defined by the inner casing **212**. The rotating member **210** may include a rod **235** that extends laterally outward and is in sliding engagement with a cam-actuating surface **234** of a locking member **246**, which is fixedly engaged to a reciprocating member **220**. As such, rotation of the rotating member **210** in either the clockwise or counter-clockwise direction causes the rod **235** to travel along the cam-actuating surface **234** of the locking member **246** and drive the reciprocating member **220** in either the distal or proximal directions **C** and **D** along longitudinal axis **500**.

The cam-actuating surface **234** of the locking member **246** may have a U-shaped configuration that permits the rod **235** to travel between an open end **250** and a closed end **252** defined by the cam-actuating surface **234**. Travel of the rod **235** between the open and closed ends **250**, **252** causes the reciprocating member **220** to move in either the distal and proximal longitudinal directions **C** and **D** as described above.

In some embodiments, the surgical instrument handle **102** may be adapted for use with any type of surgical instrument **100** in which the travel of a first structural member along the cam-actuating surface defined by a second structural member drives a third structural member to move in a longitudinal direction for effecting an operation of the surgical instrument **100**. For example, the surgical instrument **100** may be a laser probe disposed within a reciprocating member such that the reciprocating member can be retracted or extended relative to the laser probe using the motion imparted by the cam-actuating surface of a second structural member to retract or extend the reciprocating member relative to the laser probe. It is also contemplated that the surgical instrument handle **202** may be adapted for use with any type of surgical instrument **200** in which the travel of a first structural member along the cam-actuating surface defined by a second structural member drives a third structural member to move in a longitudinal direction for effecting an operation of the surgical instrument **200**. For example, the surgical instrument **200** may be a laser probe disposed within a reciprocating member such that the reciprocating member can be retracted or extended relative to the laser probe using the motion imparted by the cam-actuating surface of a second structural member to retract or extend the reciprocating member relative to the laser probe. In other embodiments, travel of a first structural member along the cam-actuating surface of a second structural member may cause a third structural member to remotely deploy the surgical instrument **100**, for example a stent (not shown), such that the reciprocating member is retracted by the travel of the first structural element along the cam-actuated surface that drives the reciprocating member to retract and deploy the stent. In some embodiments, the interaction of a first structural element with the cam-actuating surface defined by a second structural element may produce a mechanical action in a third structural element for operating surgical forceps or scissors in a cutting action. In some embodiments, the travel of a first structural element along the cam-actuating surface of a second structural element may produce a lateral action, a sliding action, a levering action, a cutting action, and/or a reciprocating action in a third structural element operatively engaged to the second structural element.

## Claims

1. A surgical instrument handle (102) comprising:
a handle body (106);
a rotating member (110) pivotally engaged to the handle body (106) for rotation in first or second rotational directions, wherein the rotating member (110) defines a cam-actuating surface (134);
a locking member (146) configured to engage the cam-actuating surface (134) of the rotating member (110); and
a reciprocating member (120) engaged to the locking member (146), wherein a rotational action of the rotating member (110) in a first rotational direction causes lateral movement of the reciprocating member (120) in a first longitudinal direction as the locking member (146) travels along the cam-actuating surface(134) of the rotating member (110) and a rotational action of the rotating member (110) in a second rotational direction causes lateral movement of the reciprocating member (120) in a second longitudinal direction as the locking member (146) travels along the cam-actuating surface (134) of the rotating member (110);
wherein the cam-actuating surface (134) is defined along an interior surface (182) of the rotating member (110), wherein the interior surface (182) defines a slot (134) having a first end (155) and a second end (157), wherein the cam-actuating surface (134) is configured to produce a cam action by the locking member (146) as the locking member (146) moves between the first end (155) and the second end (157) of the cam-actuating surface that (134) translates rotational movement of the rotating member (110) to longitudinal movement of the reciprocating member (120).

2. The handle (102) of claim 1, wherein the cam-actuating surface (134) of the rotating member (110) is configured to maintain a tangential angle at the point of contact between the locking member (146) and the cam-actuating surface of the rotating member of between 35-50 degrees.

3. The handle (102) of claim 1, wherein the locking member (146) is configured to engage the reciprocating member (120) such that the relative motion between the locking member (146) and the cam-actuating surface (134) of the rotating member (110) causes the rotational movement of the rotating member (110) to be translated to lateral movement of the reciprocating member (120).

4. The handle (102) of claim 1, further comprising:
a first hollow tube (118) disposed within the reciprocating member (120), wherein the first hollow tube (118) is made from a memory-shaped material; and
a first wire (122) coaxially disposed within the first hollow tube (118), wherein the first wire (122) is an optical fiber.

5. The handle (102) of claim 1, wherein the rotating member (110) defines a conduit (180) configured to receive a pivot pin (136) for permitting the rotating member (110) to rotate in the first rotational direction or rotate in the second rotational direction.

6. The handle (102) of claim 1, wherein the rotating member (110) defines a gripping surface comprising a plurality of protrusions (150).

7. The handle (102) of claim 1, wherein the rotating member (110) defines a distal clearing notch (138) and a proximal clearing notch (140) for allowing the rotating member (110) to be moved to a distal-most position or a proximal-most position, respectively, without interference from the handle body (106).

8. The handle (102) of claim 1, further comprising:
a second locking member (142) secured to the first hollow tube (118) for fixing the first hollow tube (118) in a stationary position such that the reciprocating member (120) moves relative to the stationary position of the first hollow tube (118).

9. The handle (102) of claim 1, wherein the locking member (146) defines a longitudinal conduit which is configured to operatively engage the reciprocating member (120).

10. The handle (102) of claim 1, wherein the handle body (106) comprises an inner casing (112) and an outer casing (116), the inner casing (112) being coupled to the outer casing (116) at a distal end of the inner casing (112) and a rear casing (114) coupled to a proximal end of the inner casing (112).

11. The handle (102) of claim 1, wherein the rotating member (110) is a wheel or a lever.

## Patentansprüche

1. Handgriff (102) für ein chirurgisches Instrument, umfassend:
einen Handgriffkörper (106),
ein Drehelement (110), das zur Drehung in einer ersten oder zweiten Drehrichtung mit dem Handgriffkörper (106) schwenkbar in Eingriff steht, wobei das Drehelement (110) eine Nockenbetätigungsfläche (134) definiert,
ein Verriegelungselement (146), das ausgebildet ist, mit der Nockenbetätigungsfläche (134) des Drehelements (110) in Eingriff zu treten, und
ein wechselbewegliches Element (120), das mit dem Verriegelungselement (146) in Eingriff steht, wobei ein Drehvorgang des Drehelements (110) in einer ersten Drehrichtung eine seitliche Bewegung des wechselbeweglichen Elements (120) in einer ersten Längsrichtung bewirkt, wenn sich das Verriegelungselement (146) entlang der Nockenbetätigungsfläche (134) des Drehelements (110) bewegt, und ein Drehvorgang des Drehelements (110) in einer zweiten Drehrichtung eine seitliche Bewegung des wechselbeweglichen Elements (120) in einer zweiten Längsrichtung bewirkt, wenn sich das Verriegelungselement (146) entlang der Nockenbetätigungsfläche (134) des Drehelements (110) bewegt,
wobei die Nockenbetätigungsfläche (134) entlang einer Innenfläche (182) des Drehelements (110) definiert ist, wobei die Innenfläche (182) einen Schlitz (134) mit einem ersten Ende (155) und einem zweiten Ende (157) definiert, wobei die Nockenbetätigungsfläche (134) ausgebildet ist, eine Nockenwirkung durch das Verriegelungselement (146) zu erzeugen, wenn sich das Verriegelungselement (146) zwischen dem ersten Ende (155) und dem zweiten Ende (157) der Nockenbetätigungsfläche bewegt, die (134) Drehbewegungen des Drehelements (110) in Längsbewegungen des wechselbeweglichen Elements (120) umwandelt.

2. Handgriff (102) nach Anspruch 1, wobei die Nockenbetätigungsfläche (134) des Drehelements (110) ausgebildet ist, einen Tangentialwinkel zwischen 35 und 50 Grad am Kontaktpunkt zwischen dem Verriegelungselement (146) und der Nockenbetätigungsfläche des Drehelements beizubehalten.

3. Handgriff (102) nach Anspruch 1, wobei das Verriegelungselement (146) ausgebildet ist, mit dem wechselbeweglichen Element (120) so in Eingriff zu treten, dass die relative Bewegung zwischen dem Verriegelungselement (146) und der Nockenbetätigungsfläche (134) des Drehelements (110) eine Umwandlung der Drehbewegung des Drehelements (110) in eine seitliche Bewegung des wechselbeweglichen Elements (120) bewirkt.

4. Handgriff (102) nach Anspruch 1, ferner umfassend:
ein erstes Hohlrohr (118), das innerhalb des wechselbeweglichen Elements (120) angeordnet ist, wobei das erste Hohlrohr (118) aus einem Formgedächtnismaterial besteht, und
einen ersten Draht (122), der koaxial innerhalb des ersten Hohlrohrs (118) angeordnet ist, wobei es sich bei dem ersten Draht (122) um einen Lichtwellenleiter handelt.

5. Handgriff (102) nach Anspruch 1, wobei das Drehelement (110) einen Kanal (180) definiert, der ausgebildet ist, einen Schwenkbolzen (136) aufzunehmen, um dem Drehelement (110) eine Drehung in der ersten Drehrichtung oder in der zweiten Drehrichtung zu ermöglichen.

6. Handgriff (102) nach Anspruch 1, wobei das Drehelement (110) eine Greiffläche definiert, die eine Vielzahl von Vorsprüngen (150) umfasst.

7. Handgriff (102) nach Anspruch 1, wobei das Drehelement (110) eine distale Freigabekerbe (138) und eine proximale Freigabekerbe (140) definiert, um dem Drehelement (110) zu ermöglichen, ohne Behinderung durch den Handgriffkörper (106) in eine maximal-distale Position bzw. eine maximal-proximale Position bewegt zu werden.

8. Handgriff (102) nach Anspruch 1, ferner umfassend:
ein an dem ersten Hohlrohr (118) befestigtes zweites Verriegelungselement (142) zum Fixieren des ersten Hohlrohrs (118) in einer ortsfesten Position, so dass sich das wechselbewegliche Element (120) relativ zu der ortsfesten Position des ersten Hohlrohrs (118) bewegt.

9. Handgriff (102) nach Anspruch 1, wobei das Verriegelungselement (146) einen Längskanal definiert, der ausgebildet ist, mit dem wechselbeweglichen Element (120) in Wirkeingriff zu treten.

10. Handgriff (102) nach Anspruch 1, wobei der Handgriffkörper (106) ein inneres Gehäuse (112) und ein äußeres Gehäuse (116) umfasst, wobei das innere Gehäuse (112) mit dem äußeren Gehäuse (116) an einem distalen Ende des inneren Gehäuses (112) verbunden ist, sowie ein rückwärtiges Gehäuse (114) umfasst, das mit einem proximalen Ende des inneren Gehäuses (112) verbunden ist.

11. Handgriff (102) nach Anspruch 1, wobei es sich bei dem Drehelement (110) um ein Rad oder einen Hebel handelt.

## Revendications

1. Manche d'instrument chirurgical (102) comprenant :
un corps de manche (106) ;
un élément rotatif (110) en prise de manière pivotante avec le corps de manche (106) en vue d'une rotation dans une première ou une seconde direction de rotation, dans lequel l'élément rotatif (110) définit une surface de mise en marche à came (134) ;
un élément de verrouillage (146) configuré pour venir en prise avec la surface de mise en marche à came (134) de l'élément rotatif (110) ; et
un élément à mouvement alternatif (120) en prise avec l'élément de verrouillage (146), dans lequel une action de rotation de l'élément rotatif (110) dans une première direction de rotation provoque un mouvement latéral de l'élément à mouvement alternatif (120) dans une première direction longitudinale tandis que l'élément de verrouillage (146) se déplace le long de la surface de mise en marche à came (134) de l'élément rotatif (110) et une action de rotation de l'élément rotatif (110) dans une seconde direction de rotation provoque un mouvement latéral de l'élément à mouvement alternatif (120) dans une seconde direction longitudinale tandis que l'élément de verrouillage (146) se déplace le long de la surface de mise en marche à came (134) de l'élément rotatif (110) ;
dans lequel la surface de mise en marche à came (134) est définie le long d'une surface intérieure (182) de l'élément rotatif (110), dans lequel la surface intérieure (182) définit une fente (134) ayant une première extrémité (155) et une seconde extrémité (157), dans lequel la surface de mise en marche à came (134) est configurée pour produire une action de came par l'élément de verrouillage (146) tandis que l'élément de verrouillage (146) se déplace entre la première extrémité (155) et la seconde extrémité (157) de la surface de mise en marche à came (134) qui convertit un mouvement rotatif de l'élément rotatif (110) en un mouvement longitudinal de l'élément à mouvement alternatif (120).

2. Manche (102) selon la revendication 1, dans lequel la surface de mise en marche à came (134) de l'élément rotatif (110) est configurée pour conserver un angle tangentiel au point de contact entre l'élément de verrouillage (146) et la surface de mise en marche à came de l'élément rotatif compris entre 35 à 50 degrés.

3. Manche (102) selon la revendication 1, dans lequel l'élément de verrouillage (146) est configuré pour venir en prise avec l'élément à mouvement alternatif (120) de telle sorte que le mouvement relatif entre l'élément de verrouillage (146) et la surface de mise en marche à came (134) de l'élément rotatif (110) provoque le mouvement rotatif de l'élément rotatif (110) qui doit être converti en un mouvement latéral de l'élément à mouvement alternatif (120).

4. Manche (102) selon la revendication 1, comprenant en outre :
un premier tube creux (118) disposé à l'intérieur de l'élément à mouvement alternatif (120), dans lequel le premier tube creux (118) est constitué d'un matériau à mémoire de forme ; et
un premier fil (122) disposé de manière coaxiale à l'intérieur du premier tube creux (118), dans lequel le premier fil (122) est une fibre optique.

5. Manche (102) selon la revendication 1, dans lequel l'élément rotatif (110) définit un conduit (180) configuré pour recevoir un axe de pivotement (136) afin de permettre à l'élément rotatif (110) de tourner dans la première direction de rotation ou de tourner dans la seconde direction de rotation.

6. Manche (102) selon la revendication 1, dans lequel l'élément rotatif (110) définit une surface de prise comprenant une pluralité de saillies (150).

7. Manche (102) selon la revendication 1, dans lequel l'élément rotatif (110) définit une encoche de dégagement distale (138) et une encoche de dégagement proximale (140) pour permettre à l'élément rotatif (110) d'être déplacé vers une position la plus distale ou une position la plus proximale, respectivement, sans interférence du corps de manche (106).

8. Manche (102) selon la revendication 1, comprenant en outre :
un second élément de verrouillage (142) fixé au premier tube creux (118) pour fixer le premier tube creux (118) dans une position stationnaire de telle sorte que l'élément à mouvement alternatif (120) se déplace par rapport à la position stationnaire du premier tube creux (118).

9. Manche (102) selon la revendication 1, dans lequel l'élément de verrouillage (146) définit un conduit longitudinal qui est configuré pour venir en prise de manière fonctionnelle avec l'élément à mouvement alternatif (120).

10. Manche (102) selon la revendication 1, dans lequel le corps de manche (106) comprend un boîtier intérieur (112) et un boîtier extérieur (116), le boîtier intérieur (112) étant couplé avec le boîtier extérieur (116) à une extrémité distale du boîtier intérieur (112) et un boîtier arrière (114) couplé avec une extrémité proximale du boîtier intérieur (112).

11. Manche (102) selon la revendication 1, dans lequel l'élément rotatif (110) est une roue ou un levier.
